# EUROPEAN PATENT APPLICATION

(11) **EP 1 876 177 A2**
(43) Date of publication of application: **09.01.2008**
(21) Application number: 07019781.9
(22) Date of filing: 31.01.2005
(51) Int. Cl.: C07D 333/16, C07D 333/24, C09K 19/34, C09K 19/38, C09K 19/54, C08G 61/12

(54) **Reactive mesogenic charge transport compounds**

(30) Priority: 25.02.2004 US 547068 P; 11.03.2004 EP 04005797
(62) Divisional of application: 05701266.8
(71) Applicant: Merck Patent GmbH, 64293 Darmstadt (DE)
(72) Inventor: Heeney, Martin, Southampton SO14 6TQ (GB); Zhang, Weimin, Southampton SO14 6TQ (GB); Tierney, Steven, Southampton SO16 4BS (GB); Sparrowe, David, Bournemouth BH6 3BB (GB); Shkunov, Maxim, Southampton SO16 6SX (GB); McCulloch, Ian, Southampton SO53 4LG (GB)

(57) **Abstract**

The invention relates to new reactive mesogenic compounds with charge transport properties comprising at least two thiophene groups, their use as semiconductors or charge transport materials, in optical, electro-optical or electronic devices like for example liquid crystal displays, optical films, organic field effect transistors (FET or OFET) for thin film transistor liquid crystal displays and integrated circuit devices such as RFID tags, electroluminescent devices in flat panel displays, and in photovoltaic and sensor devices, and to a field effect transistor, light emitting device or ID tag comprising the reactive mesogenic charge transport compounds.

## Description

### Field of Invention

The invention relates to new reactive mesogenic compounds with charge transport properties comprising at least two thiophene groups. The invention further relates to their use as semiconductors or charge transport materials, in optical, electro-optical or electronic devices like for example liquid crystal displays, optical films, organic field effect transistors (FET or OFET) for thin film transistor liquid crystal displays and integrated circuit devices such as RFID tags, electroluminescent devices in flat panel displays, and in photovoltaic and sensor devices. The invention further relates to a field effect transistor, light emitting device or ID tag comprising the reactive mesogenic charge transport compounds.

### Background and Prior Art

Organic materials have recently shown promise as the active layer in organic based thin film transistors and organic field effect transistors [see H. E. Katz, Z. Bao and S. L. Gilat, Acc. Chem. Res., 2001, 34, 5, 359]. Such devices have potential applications in smart cards, security tags and the switching element in flat panel displays. Organic materials are envisaged to have substantial cost advantages over their silicon analogues if they can be deposited from solution, as this enables a fast, large-area fabrication route.

The performance of the device is principally based upon the charge carrier mobility of the semi-conducting material and the current on/off ratio, so the ideal semiconductor should have a low conductivity in the off state, combined with a high charge carrier mobility (> 1 x 10⁻³ cm² V⁻¹ s⁻¹). In addition, it is important that the semi-conducting material is relatively stable to oxidation i.e. it has a high ionisation potential, as oxidation leads to reduced device performance.

A known compound which has been shown to be an effective p-type semiconductor for OFETs is pentacene [see S. F. Nelson, Y. Y. Lin, D. J. Gundlach and T. N. Jackson, Appl. Phys. Lett., 1998, 72, 1854]. When deposited as a thin film by vacuum deposition, it was shown to have carrier mobilities in excess of 1 cm² V⁻¹ s⁻¹ with very high current on/off ratios greater than 10⁶. However, vacuum deposition is an expensive processing technique that is unsuitable for the fabrication of large-area films.

Regular poly(3-hexylthiophene) has been reported with charge carrier mobility between 1 x 10⁻⁵ and 4.5 x 10⁻² cm² V⁻¹ s⁻¹, but with a rather low current on/off ratio between 10 and 10³ [see Z. Bao et al., Appl. Phys. Lett. 1997, 78, 2184]. In general, poly(3-alkylthiophenes) show improved solubility and are able to be solution processed to fabricate large area films. However, poly(3-alkylthiophenes) have relatively low ionisation potentials and are susceptible to doping in air [see H. Sirringhaus et al., Adv. Solid State Phys. 1999, 39, 101].

It was an aim of the present invention to provide new organic materials for use as semiconductors or charge transport materials, which are easy to synthesise, have high charge mobility and good processability. The materials should be easily processable to form thin and large-area films for use in semiconductor devices. Another aim as to extend the pool of semiconducting materials available to the expert. Other aims of the invention are immediately evident to those skilled in the art from the following description.

It was found that the above aims can be achieved by providing reactive mesogenic compounds according to the present invention. They consist of a central mesogenic core comprising two or more thiophene rings, and optionally one or more phenylene rings that form a conjugated system together with the thiophene rings, said mesogenic core being linked, optionally via a spacer group, to one or more reactive groups. The compounds can induce or enhance liquid crystal phases or are liquid crystalline themselves. They can be oriented in their mesophase and the polymerisable group can be polymerised or crosslinked in situ to form polymer films with a high degree of order, thus yielding improved semiconductor materials with high stability and high charge carrier mobility.

A further aspect of the invention relates to liquid crystal polymers, in particular liquid crystal side chain polymers obtained from the reactive mesogenic compounds according to the present invention, which are then further processed e.g. from solution as thin layers for use in semiconductor devices.

Reactive mesogenic compounds for semiconducting applications have been described in WO 03/006468 A2, EP 1 275 650 A2, EP 1 275 652 A2, EP 1 279 690 A1, EP 1 279 691 A1, EP 1 279 689 A2, EP 1 284 258 A2, EP 1 318 185 A1, EP 1 300 430 A1, EP 1 357 163 A1, EP 1 398 336 A1 and in I. McCulloch et al., J. Mater. Chem. 2003, Vol. 13, p. 2436-2444. However, compounds according to the present invention have not been disclosed.

### Definition of Terms

The term 'liquid crystal or mesogenic material' or 'liquid crystal or mesogenic compound' should denote materials or compounds comprising one or more rod-shaped, board-shaped or disk-shaped mesogenic groups, i.e. groups with the ability to induce liquid crystal phase behaviour. Liquid crystal compounds with rod-shaped or board-shaped groups are also known in the art as 'calamitic' liquid crystals. Liquid crystal compounds with a disk-shaped group are also known in the art as 'discotic' liquid crystals. The compounds or materials comprising mesogenic groups do not necessarily have to exhibit a liquid crystal phase themselves. It is also possible that they show liquid crystal phase behaviour only in mixtures with other compounds, or when the mesogenic compounds or materials, or the mixtures thereof, are polymerised.

The term 'reactive group' or 'reactive compound' means compounds or groups that are capable of participating in a polymerisation reaction, like radicalic or ionic polymerisation from unsaturated functionality, or a polyaddition or polycondensation reaction, as well as compounds or groups that are capable of being grafted for example by a condensation or addition reaction to a reactive polymer backbone in a polymeranaloguous reaction.

The term 'film' includes self-supporting, i.e. free-standing, films that show more or less pronounced mechanical stability and flexibility, as well as coatings or layers on a supporting substrate or between two substrates.

### Summary of the Invention

The invention relates to compounds of formula I

P-Sp-X-A¹-(Z¹-A²)ₘ-R I

wherein
- P: is a polymerisable or reactive group,
- Sp: is a spacer group or a single bond,
- X: is a linkage group or a single bond,
- A¹ and A²: are independently of each other 1,4-phenylene or thiophene-2,5-diyl, all of which are optionally substituted with one or more groups R¹,
- R¹: has one of the meanings of R,
- Z¹: is are independently of each other -O-, -S-, -CO-, -COO-, -OCO-, -O-CO-O-, -CO-NR⁰-, -NR⁰-CO-, -O-CO-NR⁰-, -NR⁰-CO-O-, -NR⁰-CO-NR⁰-,-OCH₂-, -CH₂O-, -SCH₂-, -CH₂S-, -CF₂O-, -OCF₂-, -CF₂S-, -SCF₂-, -CH₂CH₂-, -CF₂CH₂-, -CH₂CF₂-, -CF₂CF₂-, -CH=N-, -N=CH-, -N=N-, -CH=CR⁰-, -CY¹=CY²-, -C≡C-, -CH=CH-COO-, -OCO-CH=CH- or a single bond,
- R⁰ and R⁰⁰: are independently of each other H or alkyl with 1 to 12 C-atoms,
- Y¹ and Y²: are independently of each other H, F, Cl or CN,
- R: is H, halogen, CN, NO₂, NCS, SF₅, Sn(R')₃, SiR'R"R"' straight chain, branched or cyclic alkyl with 1 to 30 C-atoms, which is unsubstituted, mono- or polysubstituted by F, Cl, Br, I or CN, and wherein one or more non-adjacent CH₂ groups are optionally replaced, in each case independently from one another, by -O-, -S-, -NH-, -NR⁰-, -SiR'R"-, -CO-, -COO-, -OCO-, -O-CO-O-, -CO-NR⁰-, -NR⁰-CO-, -O-CO-NR⁰-, -NR⁰-CO-O-, -NR⁰-CO-NR⁰-, -S-CO-, -CO-S-, -CY¹=CY²- or -C≡C- in such a manner that O and/or S atoms are not linked directly to one another, an aromatic or heteroaromatic group, or P-Sp-X,
- R', R" and R"': are independently of each other alkyl with 1 to 12 C-atoms, and
- m: is 1, 2, 3, 4 or 5,
with the provisos that
a) at least two of A¹ and A² denote thiophene-2,5-diyl that is optionally substituted,
b) A¹ and A² are different from 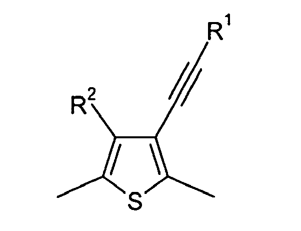 wherein R² is R¹ or -C≡C-R¹ and the groups R¹ have independently of each other one of the meanings of R given above, and
c) compounds wherein A¹-(Z¹-A²)ₘ is 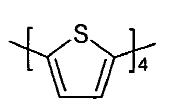 and R is P-Sp-X are excluded.

The invention also relates to the use of the compounds of formula I as semiconductors or charge transport materials, in particular in optical, electro-optical or electronic devices, like for example in field effect transistors as components of integrated circuitry, as thin film transistors in flat panel display applications or RFID tags, or in semi-conducting components for organic light emitting diode (OLED) applications such as electroluminescent displays or backlights of flat panel displays, for photovoltaic or sensor devices, or as light-modulating components for liquid crystal displays, optical films or other optical or electrooptical devices.

The invention also relates to a field effect transistor, for example as a component of integrated circuitry, as a thin film transistor in flat panel display applications, or in an RFID tag, comprising one or more compounds according to the present invention.

The invention also relates to a semi-conducting component, for example in OLED applications like electroluminescent displays or backlights of flat panel displays, in photovoltaic or sensor devices, comprising one or more according to the present invention.

### Detailed Description of the Invention

The reactive mesogenic compounds according to the present invention provide several advantages over prior art materials
- by adding substituent chains and other groups to the mesogenic core they can be made more soluble, thus being suitable for spin coating or solution coating techniques, rather than vacuum deposition, to prepare thin films for use e.g. in electronic devices such as transistors,
- they can be made mesogenic or liquid crystalline, thus exhibiting a higher degree of order that leads to particularly high charge carrier mobility, in particular when being aligned in their mesophase into macroscopically ordered orientation
- their macroscopic mesophase properties can be frozen in by in situ polymerisation,
- they combine the properties of a semi-conducting material with those of a mesogenic material to give novel materials with a rigid, planar conjugated core and a flexible chain to increase solubility and to decrease the melting point, which show high charge carrier mobility when being aligned in their mesophase.

The inventive reactive mesogenic compounds are useful as charge transport semiconductors, in that they have high carrier mobilities. In particular, the introduction of side groups to the mesogenic core improves their solubility and therefore their solution processability. In the compounds according to the present invention, the mesogenic core comprises one or more thiophene groups. They are therefore particularly useful as semiconductors or charge transport materials, as they can be processed while in the highly ordered mesophase morphology, and readily aligned by conventional techniques in a preferred direction. Both smectic and nematic mesophase ordering allows close packing of molecular pi-electron systems, which maximises intermolecular charge transfer which occurs through a hopping mechanism between adjacent molecules. This ordered, and oriented microstructure can be permanently "frozen-in" by polymerising the mesogens, which can also create a structure with long range order, or "monodomain". Formation of a monodomain also maximises charge transfer by eliminating charge trap sites at grain boundaries, while the polymerisation also improves the mechanical properties of the film. Further, by cross-linking the mesogens, a highly stable structure results, which has an additional advantage of being impervious to subsequent processing solvents during device fabrication, thus allowing a wider range of solvents to be used in deposition of the next layer of the device by solution techniques. In addition, it is often observed that this cross-linking further densifies the film, leading to smaller intermolecular distances and improved charge transport.

It is also possible to copolymerise the compounds of the present invention with other mesogenic or liquid crystal monomers that are known from prior art, or with other reactive compounds of the present invention, in order to induce or enhance liquid crystal phase behaviour.

Thus, another aspect of the invention relates to a reactive liquid crystal mixture comprising one or more reactive mesogenic compounds of the present invention, and optionally comprising one or more further reactive compounds, which are optionally also mesogenic or liquid crystalline.

Particularly preferred are reactive mesogenic compounds of the present invention, or mixtures comprising one or more reactive mesogenic compounds of the present invention, that exhibit a liquid crystal phase, especially a nematic and/or smectic liquid crystal phase.

Another aspect of invention relates to an anisotropic polymer film with charge transport properties obtainable from a reactive liquid crystal mixture as defined above that is aligned in its liquid crystal phase into macroscopically ordered orientation and polymerised or cross-linked to fix the oriented state.

Another aspect of the invention relates to a liquid crystal side chain polymer (SCLCP) obtained from a reactive liquid crystal material as defined above by polymerisation or polymeranaloguous reaction. Particularly preferred are SCLCPs obtained from one or more reactive mesogenic compounds or mixtures comprising them as described above.

Another aspect of the invention relates to an SCLCP obtained from one or more reactive mesogenic compounds or mixtures as defined above, by copolymerisation or polymeranaloguous reaction together with one or more additional mesogenic or non-mesogenic comonomers.

Side chain liquid crystal polymers or copolymers (SCLCPs), in which the semiconducting component is located as a pendant group, separated from a flexible backbone by an aliphatic spacer group, offer the possibility to obtain a highly ordered lamellar like morphology. This structure consists of closely packed conjugated aromatic mesogens, in which very close (typically < 4 A) pi-pi stacking can occur. This stacking allows intermolecular charge transport to occur more easily, leading to high charge carrier mobilities. SCLCPs are advantageous for specific applications as they can be readily synthesized before processing and then e.g. be processed from solution in an organic solvent. If SCLCPs are used in solutions, they can orient spontaneously when coated onto an appropriate surface and when at their mesophase temperature, which can result in large area, highly ordered domains.

The invention also relates to the use of reactive mesogenic compounds of the present invention, or liquid crystal mixtures or polymers obtained thereof, as light-modulating component in liquid crystal displays, which may for example be switchable between two different states by an electric field, for components of liquid crystal displays, in particular optical retardation or compensation films, alignment layers or polarisers, or in other optical or electrooptical devices.

The invention also relates to a liquid crystal display, component of a liquid crystal display, in particular an optical retardation or compensation films, alignment layer or polariser, or an other optical or electrooptical device comprising reactive mesogenic compounds according to the present invention, or liquid crystal mixtures or polymer films obtained thereof.

Very preferred are compounds of formula I wherein
- two, three, four, five or six of A¹ and A² are thiophene-2,5-diyl,
- two, three or four of A¹ and A² are thiophene-2,5-diyl and two of A¹ and A² are 1,4-phenylene,
- at least one, preferably two or more of A¹ and A² are 1,4-phenylene,
- all of A¹ and A² are thiophene-2,5-diyl,
- A¹ and A² are unsubstituted thiophene or phenylene groups,
- R or R¹ is H, F, CI or straight chain, branched or cyclic alkyl with 1 to 20 C-atoms, which is unsubstituted, mono- or polysubstituted by F, Cl, Br, I or CN, and wherein one or more non-adjacent CH₂ groups are optionally replaced, in each case independently from one another, by -O-, -S-, -NH-, -NR⁰-, -SiR⁰R⁰⁰-, -CO-, -COO-, - OCO-, -OCO-O-, -S-CO-, -CO-S-, -CY¹=CY²- or -C≡C- in such a manner that O and/or S atoms are not linked directly to one another, or an aromatic or heteroaromatic group, with Y¹ and Y² being independently of one another H, F, CI or CN,
- R or R¹ is alkyl or alkoxy with 1 to 15 C atoms which is optionally mono-, poly- or perfluorinated,
- R is P-Sp-,
- X is -O-, -O-CH₂-, -CH₂-O- or a single bond,
- all groups Z¹, A¹, A² and optionally X form a conjugated system,
- Z¹ is a single bond or a conjugated link such as -CY¹=CY²- or - C≡C-,
- Z¹ is different from -CF=CF-,
- one or more, preferably all of Z¹ are a single bond,
- if all of A¹ and A² are thiophene and Z¹ is a single bond then m is 1, 2, 4 or 5,
- m is 2, 3, 4 or 5,
- m is 3 or 4.

Preferably A¹ and A² are different from 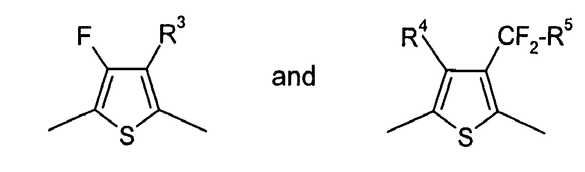 wherein
R³ is straight chain, branched or cyclic alkyl with 1 to 20 C-atoms, which is unsubstituted, mono- or poly-substituted by F, Cl, Br, I or CN, and wherein one or more non-adjacent CH₂ groups are optionally replaced, in each case independently from one another, by -O-, -S-, -NH-, -NR⁰-, -SiR⁰R⁰⁰-, -CO-, -COO-, -OCO-, -OCO-O-, -S-CO-, -COS-, -CH=CH- or -C≡C- in such a manner that O and/or S atoms are not linked directly to one another, or optionally substituted aryl or heteroaryl,
R⁴ is H, F or R³, and
R⁵ is H, straight chain, branched or cyclic alkyl with 1 to 20 C-atoms wherein one or more, but not all, H atoms are optionally replaced by F, Cl, Br, I or CN, and wherein one or more non-adjacent CH₂ groups are optionally replaced, in each case independently from one another, by - O-, -S-, -NH-, -NR⁰-, -SiR⁰R⁰⁰-, -CO-, -COO-, -OCO-, -OCO-O-, -S-CO-, -CO-S-, -CH=CH- or -C≡C- in such a manner that O and/or S atoms are not linked directly to one another, or optionally substituted aryl or heteroaryl,
with R⁰ and R⁰⁰ being as defined in formula I.

A preferred embodiment of the present invention relates to compounds wherein -A¹-(Z¹-A²)ₘ- is selected of formula II1

-Phe-(Z-Thi)ₘ₁-Z-Phe- II1

wherein m1 is 2, 3 or 4, the groups Z independently of each other have one of the meanings of Z¹ as defined above, Thi is a thiophene-2,5-diyl group and Phe is a 1,4-phenylene group, these groups being optionally substituted by one or more groups R¹ as defined above.

Another preferred embodiment of the present invention relates to compounds wherein -A¹-(Z¹-A²)ₘ- is selected of formula II2

-Thi-(Z-Thi)ₘ- II2

wherein m is 1, 2, 4 or 5 and Z and Thi are as defined in formula II1.

Further preferred groups -A¹-(Z¹-A²)ₘ- are selected of the following formulae and their mirror images

| | |
|---|---|
| -Phe-Z-Thi-Z-Thi- | II3 |
| -Thi-Z-Phe-Z-Thi- | II4 |
| -Phe-Z-Thi-Thi-Thi- | II5 |
| -Phe-Z-Phe-Z-Thi-Z-Thi- | II6 |
| -Thi-Z-Phe-Z-Phe-Z-Thi- | II7 |
| -Phe-Z-Thi-Z-Thi-Z-Thi-Z-Thi- | II8 |
| -Thi-Z-Phe-Z-Thi-Z-Thi-Z-Thi- | II9 |
| -Thi-Z-Thi-Z-Phe-Z-Thi-Z-Thi- | II10 |
| -Phe-Z-Phe-Z-Thi-Z-Thi-Z-Thi- | II11 |
| -Thi-Z-Phe-Z-Phe-Z-Thi-Z-Thi- | II12 |
| -Thi-Z-Phe-Z-Thi-Z-Phe-Z-Thi- | II13 |
| -Phe-Z-Phe-Z-Phe-Z-Thi-Z-Thi- | II14 |
| -Phe-Z-Thi-Z-Phe-Z-Thi-Z-Phe- | II15 |
| -Thi-Z-Phe-Z-Phe-Z-Phe-Z-Thi- | II16 |
| -Phe-Z-Thi-Z-Thi-Z-Thi-Z-Thi-Z-Thi- | II17 |
| -Phe-Z-Phe-Z-Thi-Z-Thi-Z-Thi-Z-Thi- | II18 |
| -Phe-Z-Phe-Z-Phe-Z-Thi-Z-Thi-Z-Thi- | II19 |
| -Phe-Z-Phe-Z-Phe-Z-Phe-Z-Thi-Z-Thi- | II20 |
| -Thi-Z-Thi-Z-Phe-Z-Phe-Z-Thi-Z-Thi- | II21 |
| -Thi-Z-Phe-Z-Phe-Z-Phe-Z-Thi-Z-Thi- | II22 |
| -Thi-Z-Phe-Z-Phe-Z-Phe-Z-Phe-Z-Thi- | II23 |
| -Phe-Z-Phe-Z-Thi-Z-Thi-Z-Phe-Z-Phe- | II24 |
| -Phe-Z-Thi-Z-Phe-Z-Phe-Z-Thi-Z-Phe- | II25 |
| -Thi-Z-Phe-Z-Thi-Z-Thi-Z-Phe-Z-Thi- | II26 |
| -Thi-Z-Thi-Z-Phe-Z-Phe-Z-Thi-Z-Thi- | II27 |

wherein Z, Thi and Phe have the meanings given above.

Especially preferably Z in formulae II1 to II27 is a single bond.

Particularly preferred are compounds of the following formulae 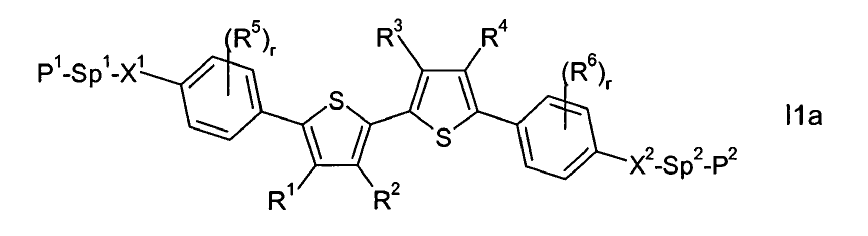 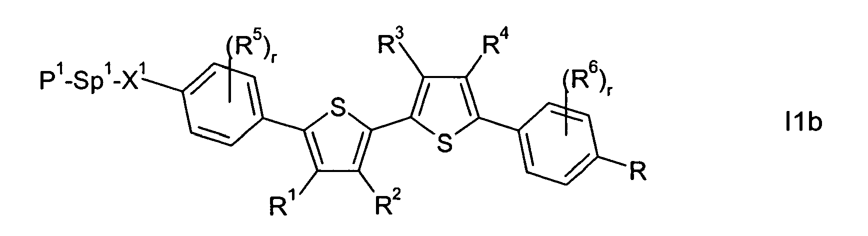 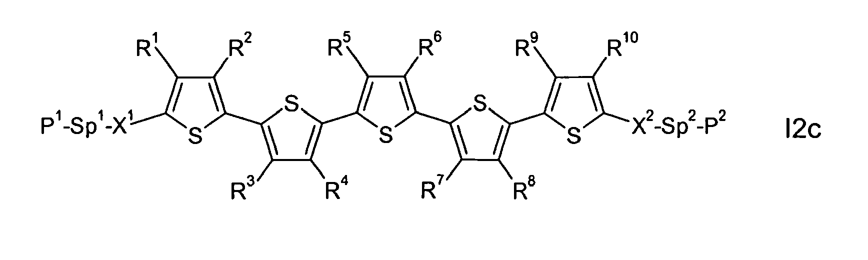 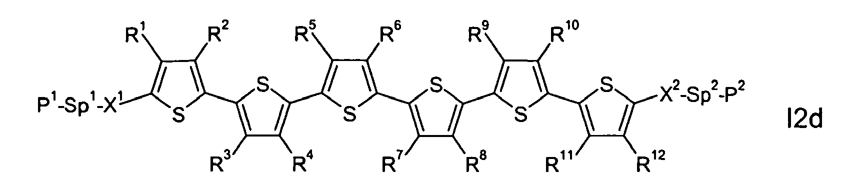 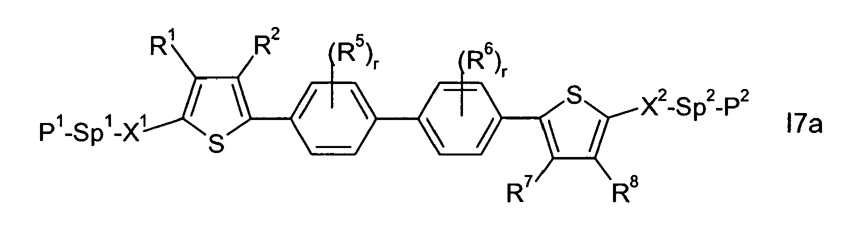 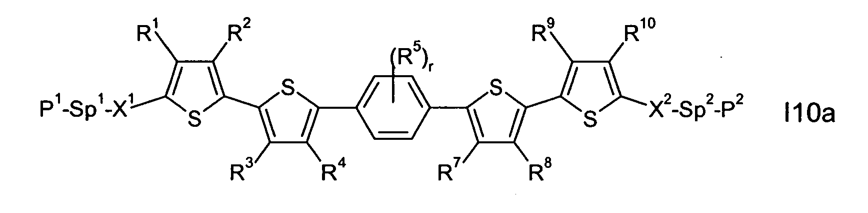 wherein
- P¹ and P²: are identical or different groups P as defined in formula I,
- Sp¹ and Sp²: are identical or different groups Sp as defined in formula I,
- X¹ and X²: are identical or different groups X as defined in formula I,
- R: is as defined in formula I,
- R¹ to R¹²: have independently of one another one of the meanings of R¹ in formula I, and are preferably H, halogen or straight-chain alkyl with 1 to 8 C-atoms that is optionally mono-, poly- or perfluorinated, and
- r: is 0, 1, 2, 3 or 4.

Very preferred are the following compounds 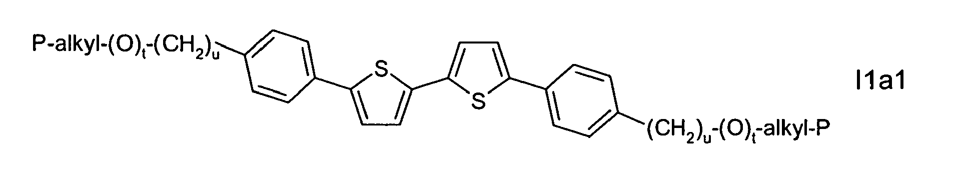 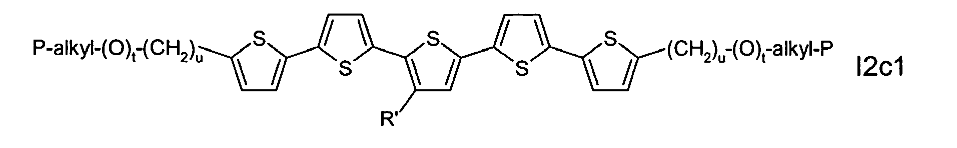 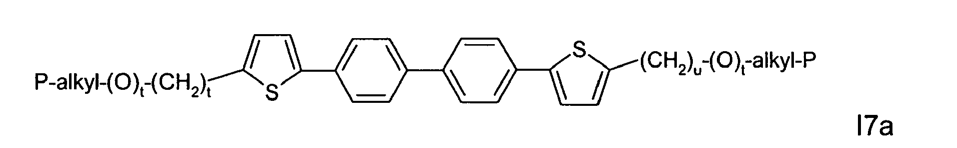 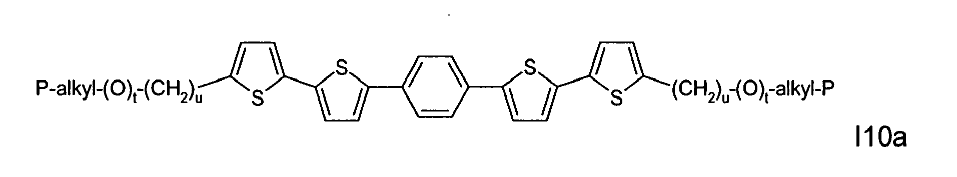 wherein "alkyl" is an alkylene group with 1 to 12, preferably 2 to 8 C-atoms, t is 0 or 1, u is 0 or 1, R' is H or optionally fluorinated C₁₋₈-alkyl and P is CH₂=CW-COO-, 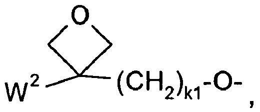 (CH₂=CH)₂CH-OCO-or (CH₂=CH-CH₂)₂N-CO-, W is H, CH₃, Cl or CN and k1 is 0 or 1. -CY¹=CY²- is preferably -CH=CH-, -CH=CF-, -CF=CH-, -CF=CF-, -CH=C(CN)- or -C(CN)=CH-.

If one of R¹ to R¹² is an alkyl or alkoxy radical, i.e. where the terminal CH₂ group is replaced by -O-, this may be straight-chain or branched. It is preferably straight-chain, has 2, 3, 4, 5, 6, 7 or 8 carbon atoms and accordingly is preferably ethyl, propyl, butyl, pentyl, hexyl, heptyl, octyl, ethoxy, propoxy, butoxy, pentoxy, hexoxy, heptoxy, or octoxy, furthermore methyl, nonyl, decyl, undecyl, dodecyl, tridecyl, tetradecyl, pentadecyl, nonoxy, decoxy, undecoxy, dodecoxy, tridecoxy or tetradecoxy, for example.

Oxaalkyl, i.e. where one CH₂ group is replaced by -O-, is preferably straight-chain 2-oxapropyl (=methoxymethyl), 2- (=ethoxymethyl) or 3-oxabutyl (=2-methoxyethyl), 2-, 3-, or 4-oxapentyl, 2-, 3-, 4-, or 5-oxahexyl, 2-, 3-, 4-, 5-, or 6-oxaheptyl, 2-, 3-, 4-, 5-, 6- or 7-oxaoctyl, 2-, 3-, 4-, 5-, 6-, 7- or 8-oxanonyl or 2-, 3-, 4-, 5-, 6-,7-, 8- or 9-oxadecyl, for example.

Halogen is preferably F or Cl.

Hetero atoms are preferably selected from N, O and S.

The polymerisable or reactive groups P, P¹ and P² are preferably selected from CH₂=CW¹-COO-, 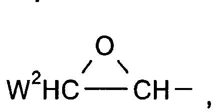 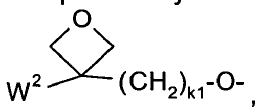 CH₂=CW²-(O)ₖ₁-, CH₃-CH=CH-O-, (CH₂=CH)₂CH-OCO-, (CH₂=CH-CH₂)₂CH-OCO-, (CH₂=CH)₂CH-O-, (CH₂=CH-CH₂)₂N-, (CH₂=CH-CH₂)₂N-CO-, HO-CW²W³-, HS-CW²W³-, HW²N-, HO-CW²W³-NH-, CH₂=CW¹-CO-NH-, CH₂=CH-(COO)ₖ₁-Phe-(O)ₖ₂-, Phe-CH=CH-, HOOC-, OCN-, and W⁴W⁵W⁶Si-, with W¹ being H, Cl, CN, phenyl or alkyl with 1 to 5 C-atoms, in particular H, Cl or CH₃, W² and W³ being independently of each other H or alkyl with 1 to 5 C-atoms, in particular methyl, ethyl or n-propyl, W⁴, W⁵ and W⁶ being independently of each other Cl, oxaalkyl or oxacarbonylalkyl with 1 to 5 C-atoms, Phe being 1,4-phenylene that is optionally substituted by one or more groups R¹ as defined above, and k₁ and k₂ being independently of each other 0 or 1.

Especially preferred groups P and P^{1,2} are CH₂=CH-COO-, CH₂=C(CH₃)-COO-, CH₂=CH-, CH₂=CH-O-, (CH₂=CH)₂CH-OCO-, (CH₂=CH)₂CH-O-, (CH₂=CH-CH₂)₂N-, (CH₂=CH-CH₂)₂N-CO- and

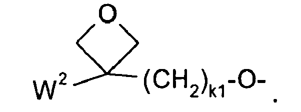

Very preferred are acrylate, methacrylate, diene, (CH₂=CH)₂CH-OCO and oxetane groups. Oxetanes produce less shrinkage upon polymerisation (cross-linking), which results in less stress development within films, leading to higher retention of ordering and fewer defects. Oxetane cross-linking also requires cationic initiator, which unlike free radical initiator is inert to oxygen.

As for the spacer group Sp, Sp¹ and Sp² all groups can be used that are known for this purpose to those skilled in the art. The spacer group Sp or Sp^{1,2} is preferably a linear or branched alkylene group having 1 to 20 C atoms, in particular 1 to 12 C atoms, in which, in addition, one or more non-adjacent CH₂ groups may be replaced by -O-, -S-, -NH-, -N(CH₃)-, -CO-, -O-CO-, -S-CO-, -O-CO-O-, -NR⁰-CO-NR⁰, -NR⁰-CO-O-, -O-CO-NR⁰-, -CO-S-, -CO-O-, -CH(halogen)-, -C(halogen)₂, -CH(CN)-, -CH=CH- or -C≡C-, or a siloxane group.

Typical spacer groups are for example -(CH₂)ₚ-, -(CH₂CH₂O)_{q} -CH₂CH₂-, -CH₂CH₂-S-CH₂CH₂- or -CH₂CH₂-NH-CH₂CH₂- or -(SiR⁰R⁰⁰-O)ₚ-, with p being an integer from 2 to 12, q being an integer from 1 to 3 and R⁰ and R⁰⁰ having the meanings given in formula I.

Preferred spacer groups are ethylene, propylene, butylene, pentylene, hexylene, heptylene, octylene, nonylene, decylene, undecylene, dodecylene, octadecylene, ethyleneoxyethylene, methyleneoxybutylene, ethylene-thioethylene, ethylene-N-methyl-iminoethylene, 1-methylalkylene, ethenylene, propenylene and butenylene for example.

The linkage group X, X¹ and X² preferably denotes -O-, -S-, -OCH₂-, -CH₂O-, -CO-, -COO-, -OCO-, -O-CO-O-, -CO-NR⁰-, -NR⁰-CO-, -O-CO-NR⁰-, -NR⁰-CO-O-, -NR⁰-CO-NR⁰-, -OCH₂-, -CH₂O-, -SCH₂-, -CH₂S-, -CH=CH-COO-, -OOC-CH=CH-, -CF₂O-, -OCF₂-, -CF₂S-, -SCF₂-, -CH₂CH₂-, -CF₂CH₂-, -CH₂CF₂-, -CF₂CF₂-, -CH=N-, -N=CH-, -N=N-, -CH=CR⁰-, -CY¹=CY²-, -C≡C- or a single bond, with R⁰, Y¹ and Y² having the meanings given above.

In a preferred embodiment, the linkage group X, X¹ or X² is an unsaturated group that is capable of forming a conjugated system, such as -CH=N-, -N=CH-, -N=N-, -CH=CR⁰-, -CY¹=CY²-, -C≡C-, or a single bond.

Further preferred are compounds with one or two groups P-Sp-X wherein Sp and/or X is a single bond.

In case of compounds with two or more groups P-Sp-X, each of the groups P, the groups Sp, and the groups X can be identical or different.

SCLCPs obtained from the inventive compounds or mixtures by polymerisation or copolymerisation have a backbone that is formed by the polymerisable group P in formula I.

The compounds of formula I can be synthesized according to or in analogy to methods that are known to the skilled in the art and are reported in the literature. Furthermore, they can be prepared according to or in analogy to the following reaction schemes.

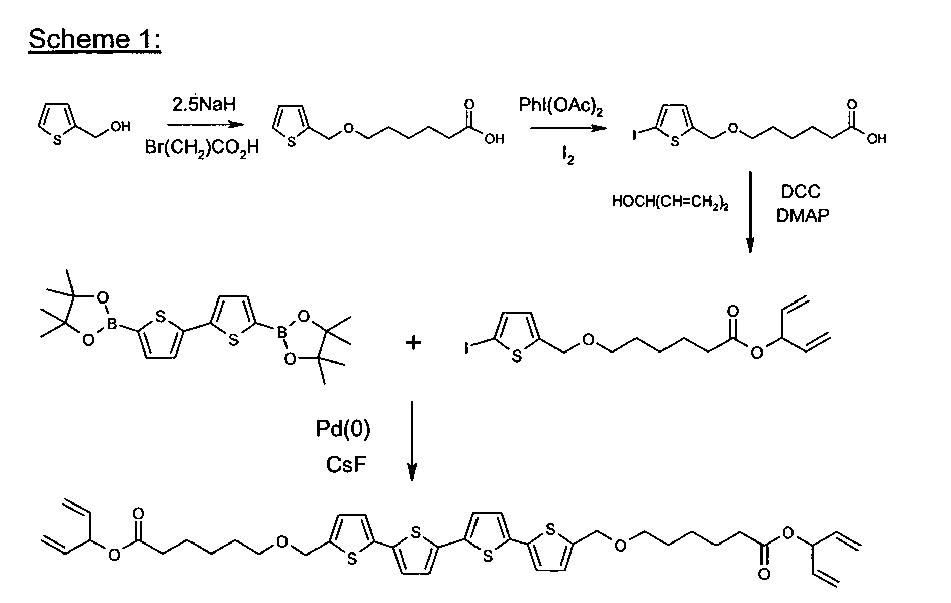

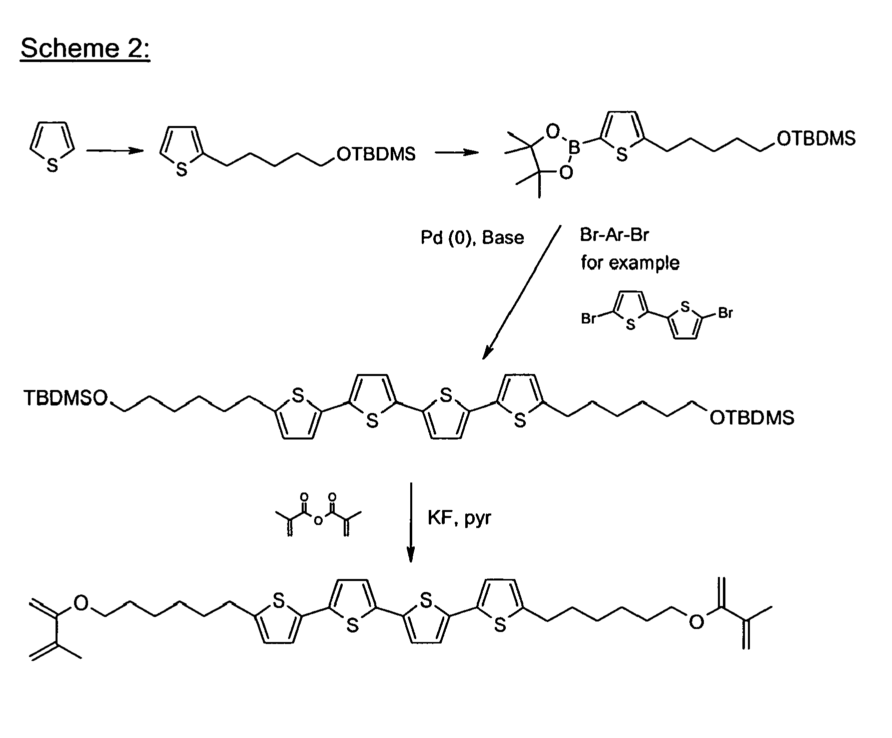

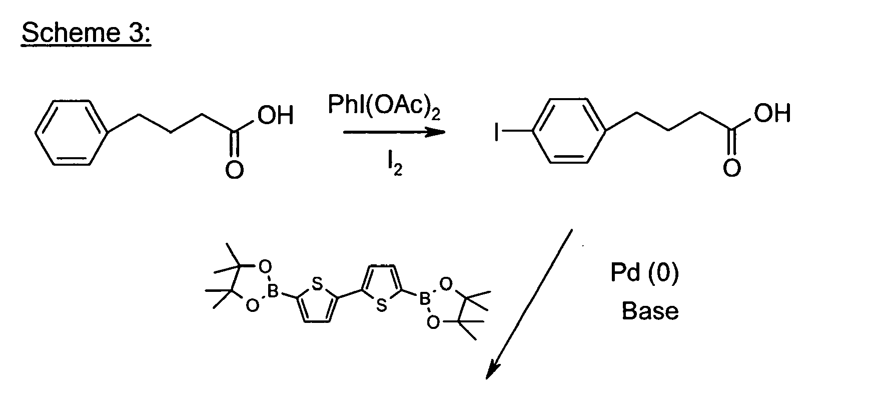

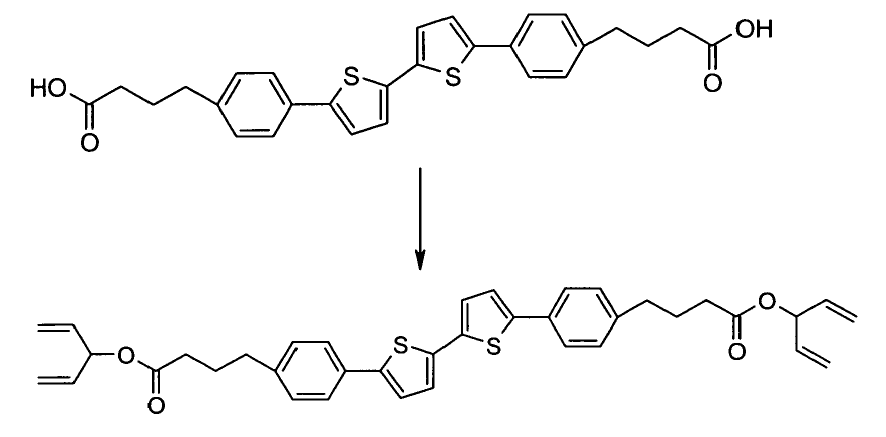

As outlined in scheme 1-3, compounds of formula I can be synthesised by transition metal catalysed cross-coupling methodologies. A convienent procedure is the Suzuki reaction involving aryl boronic acids, however the present compounds can also be synthesised by the transition metal catalysed coupling of organotins (Stille reaction), organozincs (Negishi coupling), organomagnesiums (Kumada coupling), organosilicon reagents or aryl lithiums. The polymerisable endgroups can be incorporated before the cross-coupling step (scheme 1), or afterwards (scheme 2 or 3).

The reactive mesogenic compounds of formula I are particularly useful as semiconductors or charge transport materials, as they can be aligned into uniform highly ordered orientation in their liquid crystal phase by known techniques, thus exhibiting a higher degree of order that leads to particularly high charge carrier mobility. The highly ordered liquid crystal state can be fixed by in situ polymerisation or crosslinking via the groups P to yield polymer films with high charge carrier mobility and high thermal, mechanical and chemical stability.

It is also possible to copolymerise the compounds according to the present invention with other polymerisable mesogenic or liquid crystal monomers that are known from prior art, in order to induce or enhance liquid crystal phase behaviour.

Thus, another aspect of the invention relates to a polymerisable liquid crystal material comprising one or more reactive mesogenic compounds of the present invention as described above and below comprising at least one polymerisable group, and optionally comprising one or more further polymerisable compounds, wherein at least one of the reactive mesogenic compounds of the present invention and/or the further polymerisable compounds is mesogenic or liquid crystalline.

Particularly preferred are liquid crystal materials having a nematic and/or smectic phase. For FET applications smectic materials are especially preferred. For OLED applications nematic or smectic materials are especially preferred. Especially preferred are smectic A (S_{A}) phases, furthermore highly ordered smectic phases like the S_{B}, S_{E}, S_{G} and S_{F} phase.

Another aspect of the invention relates to an anisotropic polymer film with charge transport properties obtainable from a polymerisable liquid crystal material as defined above that is aligned in its liquid crystal phase into macroscopically uniform orientation and polymerised or crosslinked to fix the oriented state.

Polymerisation is preferably carried out by in-situ polymerisation of a coated layer of the material, preferably during fabrication of the electronic or optical device comprising the inventive semiconductor material. In case of liquid crystal materials, these are preferably aligned in their liquid crystal state into homeotropic orientation prior to polymerisation, where the conjugated pi-electron systems are orthogonal to the direction of charge transport. This ensures that the intermolecular distances are minimised and hence then energy required to transport charge between molecules is minimised. The molecules are then polymerised or crosslinked to fix the uniform orientation of the liquid crystal state. Alignment and curing are carried out in the liquid crystal phase or mesophase of the material. This technique is known in the art and is generally described for example in D.J. Broer, et al., Angew. Makromol. Chem. 183, (1990), 45-66.

Alignment of the liquid crystal material can be achieved for example by treatment of the substrate onto which the material is coated, by shearing the material during or after coating, by application of a magnetic or electric field to the coated material, or by the addition of surface-active compounds to the liquid crystal material. Reviews of alignment techniques are given for example by I. Sage in "Thermotropic Liquid Crystals", edited by G. W. Gray, John Wiley & Sons, 1987, pages 75-77, and by T. Uchida and H. Seki in "Liquid Crystals - Applications and Uses Vol. 3", edited by B. Bahadur, World Scientific Publishing, Singapore 1992, pages 1-63. A review of alignment materials and techniques is given by J. Cognard, Mol. Cryst. Liq. Cryst. 78, Supplement 1 (1981), pages 1-77.

Polymerisation takes place by exposure to heat or actinic radiation. Actinic radiation means irradiation with light, like UV light, IR light or visible light, irradiation with X-rays or gamma rays or irradiation with high energy particles, such as ions or electrons. Preferably polymerisation is carried out by UV irradiation at a non-absorbing wavelength. As a source for actinic radiation for example a single UV lamp or a set of UV lamps can be used. When using a high lamp power the curing time can be reduced. Another possible source for actinic radiation is a laser, like e.g. a UV laser, an IR laser or a visible laser.

Polymerisation is preferably carried out in the presence of an initiator absorbing at the wavelength of the actinic radiation. For example, when polymerising by means of UV light, a photoinitiator can be used that decomposes under UV irradiation to produce free radicals or ions that start the polymerisation reaction. When curing polymerisable materials with acrylate or methacrylate groups, preferably a radical photoinitiator is used, when curing polymerisable materials with vinyl, epoxide and oxetane groups, preferably a cationic photoinitiator is used. It is also possible to use a polymerisation initiator that decomposes when heated to produce free radicals or ions that start the polymerisation. As a photoinitiator for radical polymerisation for example the commercially available Irgacure 651, Irgacure 184, Darocure 1173 or Darocure 4205 (all from Ciba Geigy AG) can be used, whereas in case of cationic photopolymerisation the commercially available UVI 6974 (Union Carbide) can be used.

The polymerisable material can additionally comprise one or more other suitable components such as, for example, catalysts, sensitizers, stabilizers, inhibitors, chain-transfer agents, co-reacting monomers, surface-active compounds, lubricating agents, wetting agents, dispersing agents, hydrophobing agents, adhesive agents, flow improvers, defoaming agents, deaerators, diluents, reactive diluents, auxiliaries, colourants, dyes or pigments.

Polymerisable benzodithiophenes comprising one or more groups P-Sp-X can also be copolymerised with polymerisable mesogenic compounds to induce, or, in case of mesogenic materials of formula I, enhance liquid crystal phase behaviour. Polymerisable mesogenic compounds that are suitable as comonomers are known in prior art and disclosed for example in WO 93/22397; EP 0,261,712; DE 195,04,224; WO 95/22586 and WO 97/00600.

SCLCPs can be prepared from the polymerisable compounds or mixtures according to the invention by the methods described above, or by conventional polymerisation techniques which are known to those skilled in the art, including for example radicalic, cationic or anionic polymerisation from unsaturated functionality radicalic, polyaddition or polycondensation. Polymerisation can be carried out for example as polymerisation in solution, without the need of coating and prior alignment, or polymerisation in situ.

It is also possible to form SCLCPs by grafting compounds according to the invention with a suitable reactive group, or mixtures thereof, to presynthesized isotropic or anisotropic polymer backbones in a polymeranaloguous reaction. For example, compounds with a terminal hydroxy group can be attached to polymer backbones with lateral carboxylic acid or ester groups, compounds with terminal isocyanate groups can be added to backbones with free hydroxy groups, compounds with terminal vinyl or vinyloxy groups can be added e.g. to polysiloxane backbones with Si-H groups.

It is also possible to form SCLCPs by copolymerisation or polymeranaloguous reaction from the inventive compounds together with conventional mesogenic or non mesogenic comonomers. Suitable comonomers are known to those skilled in the art. In principle it is possible to use all conventional comonomers known in the art that carry a reactive or polymerisable group capable of undergoing the desired polymer-forming reaction, like for example a polymerisable or reactive group P as defined above. Typical mesogenic comonomers are for example those mentioned in WO 93/22397; EP 0,261,712; DE 195,04,224; WO 95/22586 and WO 97/00600. Typical non mesogenic comonomers are for example alkyl mono- or diacrylates or alkyl mono-or dimethacrylates with alkyl groups of 1 to 20 C atoms, like methyl acrylate or methyl methacrylate, trimethylpropane trimethacrylate or pentaerythritol tetraacrylate.

For example, if a device is made from a polymerisable liquid crystal material by polymerisation in situ, the liquid crystal material preferably comprises one or more compounds of formula I and its preferred subformulae having one or more groups P. If a liquid crystal polymer is prepared first, for example by polymerisation in solution, and the isolated polymer is used to make the device, the polymer is preferably made from a liquid crystal material comprising one or more compounds of formula I and its preferred subformulae having one group P.

The materials of the present invention are useful as optical, electronic and semiconductor materials, in particular as charge transport materials in field effect transistors (FETs) e.g. as components of integrated circuitry, ID tags or TFT applications. Alternatively, they may be used in organic light emitting diodes (OLEDs) in electroluminescent display applications or as backlight of e.g. liquid crystal displays, as photovoltaics or sensor materials, for electrophotographic recording, and for other semiconductor applications.

Especially the oligomers and polymers according to the invention show advantageous solubility properties which allow production processes using solutions of these compounds. Thus films, including layers and coatings, may be generated by low cost production techniques e.g. spin coating. Suitable solvents or solvent mixtures comprise alkanes and/ or aromatics, especially their fluorinated derivatives.

The materials of the present invention are useful as optical, electronic and semiconductor materials, in particular as charge transport materials in field effect transistors (FETs), as photovoltaics or sensor materials, for electrophotographic recording, and for other semiconductor applications. Such FETs, where an organic semiconductive material is arranged as a film between a gate-dielectric and a drain and a source electrode, are generally known e.g. from US 5,892,244, WO 00/79617, US 5,998,804, and from the references cited in the background and prior art chapter and listed below. Due to the advantages, like low cost production using the solubility properties of the compounds according to the invention and thus the processibility of large surfaces, preferred applications of these FETs are such as integrated circuitry, TFT-displays and security applications.

In security applications, field effect transistors and other devices with semiconductive materials, like transistors or diodes, may be used for ID tags or security markings to authenticate and prevent counterfeiting of documents of value like banknotes, credit cards or ID cards, national ID documents, licenses or any product with money value, like stamps, tickets, shares, cheques etc..

Alternatively, the materials according to the invention may be used in organic light emitting devices or diodes (OLEDs), e.g. in display applications or as backlight of e.g. liquid crystal displays. Common OLEDs are realized using multilayer structures. An emission layer is generally sandwiched between one or more electron-transport and/ or hole-transport layers. By applying an electric voltage electrons and holes as charge carriers move towards the emission layer where their recombination leads to the excitation and hence luminescence of the lumophor units contained in the emission layer. The inventive compounds, materials and films may be employed in one or more of the charge transport layers and/ or in the emission layer, corresponding to their electrical and/ or optical properties. Furthermore their use within the emission layer is especially advantageous, if the compounds, materials and films according to the invention show electroluminescent properties themselves or comprise electroluminescent groups or compounds. The selection, characterization as well as the processing of suitable monomeric, oligomeric and polymeric compounds or materials for the use in OLEDs is generally known by a person skilled in the art, see e. g. Meerholz, Synthetic Materials, 111-112, 2000, 31-34, Alcala, J. Appl. Phys., 88, 2000, 7124-7128 and the literature cited therein.

According to another use, the inventive compounds, materials or films, especially those which show photoluminescent properties, may be employed as materials of light sources, e.g. of display devices such as described in EP 0 889 350 A1 or by C. Weder et al., Science, 279, 1998, 835-837.

The materials of the present invention are also useful for the preparation of optical films with anisotropic properties, like for example polarizers, optical retardation films, compensators, colour filters, polarization beam splitters, or polarization filters, which can be used for example as components of liquid crystal displays. Furthermore, they can be used as coatings e.g. for decorative or security use, as adhesives, or for the preparation of liquid crystal pigments.

A further aspect of the invention relates to both the oxidised and reduced form of the compounds and materials according to this invention. Either loss or gain of electrons results in formation of a highly delocalised ionic form, which is of high conductivity. This can occur on exposure to common dopants. Suitable dopants and methods of doping are known to those skilled in the art, e.g. from EP 0 528 662, US 5,198,153 or WO 96/21659.

The doping process typically implies treatment of the semiconductor material with an oxidating or reducing agent in a redox reaction to form delocalised ionic centres in the material, with the corresponding counterions derived from the applied dopants. Suitable doping methods comprise for example exposure to a doping vapor in the atmospheric pressure or at a reduced pressure, electrochemical doping in a solution containing a dopant, bringing a dopant into contact with the semiconductor material to be thermally diffused, and ion-implantantion of the dopant into the semiconductor material.

When electrons are used as carriers, suitable dopants are for example halogens (e.g. I₂, Cl₂, Br₂, ICI ICl₃, IBr and IF), Lewis acids (e.g. PF₅, AsF₅, SbF₅, BF₃, BCl₃, SbCl₅, BBr₃ and SO₃), protonic acids, organic acids, or amino acids (e.g. HF, HCl, HNO₃, H₂SO₄, HClO₄, FSO₃H and ClSO₃H), transition metal compounds (e.g. FeCl₃, FeOCl, Fe(ClO₄)₃, Fe(4-CH₃C₆H₄SO₃)₃, TiCl₄, ZrCl₄, HfCl₄, NbF₅, NbCl₅, TaCl₅, MoF₅, MoCl₅, WF₅, WCl₆, UF₆ and LnCl₃ (wherein Ln is a lanthanoid), anions (e.g. Cl⁻, Br⁻, I⁻, I₃⁻, HSO₄⁻, SO₄²⁻, NO₃⁻, ClO₄⁻, BF₄⁻, PF₆⁻, AsF₆⁻, SbF₆⁻, FeCl₄⁻, Fe(CN)₆³⁻, and anions of various sulfonic acids, such as aryl-SO₃⁻). When holes are used as carriers, examples of dopants are cations (e.g. H⁺, Li⁺, Na⁺, K⁺, Rb⁺ and Cs⁺), alkali metals (e.g., Li, Na, K, Rb, and Cs), alkaline-earth metals (e.g., Ca, Sr, and Ba), O₂, XeOF₄, (NO₂⁺) (SbF₆⁻), (NO₂⁺) (SbCl₆⁻), (NO₂⁺) (BF₄⁻), AgClO₄, H₂IrCl₆, La(NO₃)₃ · 6H₂O, FSO₂OOSO₂F, Eu, acetylcholine, R₄N⁺, R₄P⁺, R₆As⁺, and R₃S⁺ (wherein R is an alkyl group).

The conducting form of the compounds and materials of the present invention can be used as an organic "metal" in applications, for example, but not limited to, charge injection layers and ITO planarising layers in organic light emitting diode applications, films for flat panel displays and touch screens, antistatic films, printed conductive substrates, patterns ot tracts in electronic applications such as printed circuit boards and condensers.

The examples below serve to illustrate the invention without limiting it. In the foregoing and the following, all temperatures are given in degrees Celsius, and all percentages are by weight, unless stated otherwise. Sₓ and Sₓ₁ refer to smectic liquid crystal phases of undetermined structure.

### Example 1

Compound (4) is prepared as follows:

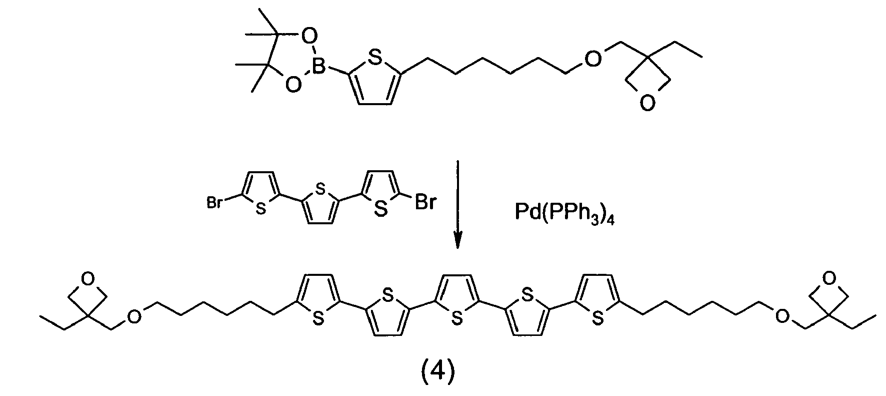

### Step 4.1:

Tetrakis(triphenylphosphine)palladium(0) (0.05 g) is added to a solution of 5',5"-dibromo-[2,2';5',2"]terthiophene (0.10g, 0.25 mmol) in dry THF (30 ml), with stirring, under nitrogen. After 20 min, 2-{5-[6-(3-Ethyl-oxetan-3-ylmethoxy) -hexyl]thiophen-2-yl}-4,4,5,5-tetramethyl-[1,3,2]dioxaborolane (0.25 g, 0.60 mmol) and a solution of potassium carbonate (0.17 g, 1.23 mmol) in water (5 ml) is added. The resultant mixture is heated at reflux for 1.5 h. After cooling, water (100 ml) is added and the precipitate filtered off, washed with water and diethyl ether, to give a red solid, which is recrystallised with toluene to offer red crystals (0.14 g, 70 %). ¹H NMR (300 MHz, CDCl₃): δ (ppm) 6.97-7.07 (m, 8H, Ar-H), 6.68 (d, *J* = 2.5 Hz, 2H, Ar-H), 4.45 (d, *J* = 5.7 Hz, 4H, OCH₂), 4.38 (d, *J* = 5.7 Hz, 4H, OCH₂), 3.52 (s, 4H, OCH₂), 3.45 (t, *J* = 6.4 Hz, 4H, OCH₂), 2.80 (t, *J* = 7.4 Hz 4H, ArCH₂), 1.35-1.78 (m, 20H, CH₂), 0.88 (t, *J* = 7.4 Hz, 6H, CH₃); ¹³C NMR (75 MHz, CDCl₃): δ (ppm) 145.5 (quat.), 136.9 (quat.), 136.0 (quat.), 135.2 (quat.), 134.5 (quat.), 125.0 (CH),124.2 (CH), 124.1 (CH), 123.6 (CH),123.5 (CH), 78.7 (OCH₂), 73.4 (OCH₂), 71.5 (OCH₂), 43.4 (quat.), 31.5 (CH₂), 30.2 (CH₂), 29.5 (CH₂), 28.9 (CH₂), 26.8 (CH₂) 25.9 (CH₂), 8.3 (CH₃). m.p. (°C) K 98 S_{X} 180 I

### Example 2 - Comparative Example

Compound (7) is prepared as follows:

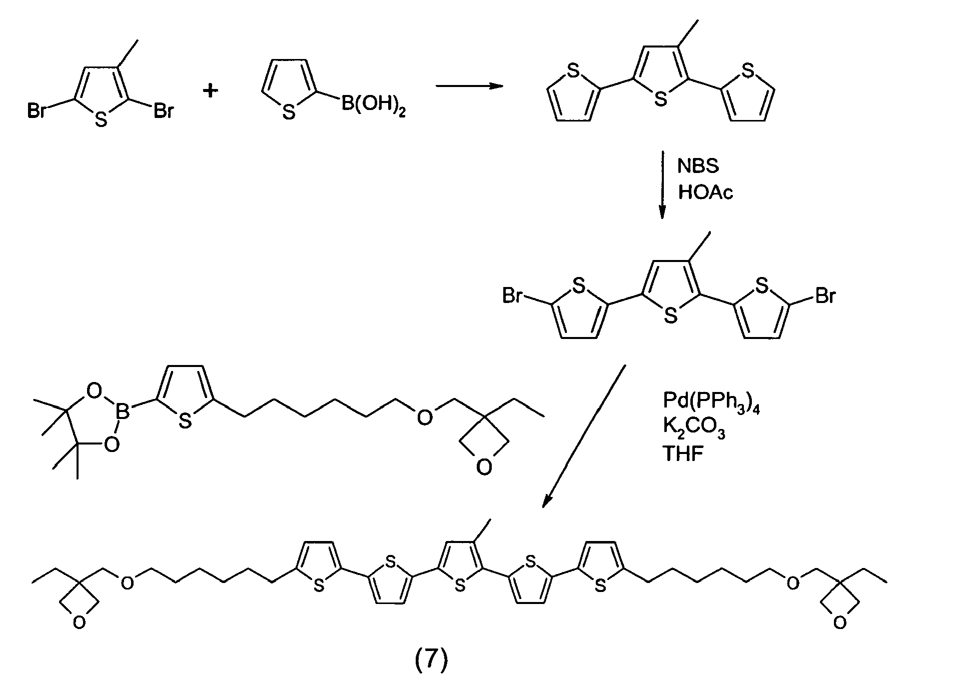

### Step 7.1: 3'-Methyl-[2,2',5',2"]terthiophene

To a stirred solution of Pd₂(dba)₃ (0.30 g, 0.33 mmol) in anhydrous THF (70 ml) is added [(t-Bu₃)PH]BF₄ (0.38 g,1.32 mmol) under N₂, with stirring. After 5 min, 2,5-dibromo-3-methylthiophene (7.0g, 27.35 mmol) is introduced and this mixture is stirred for another 10 min, followed by the addition of thiophene-2-boronic acid (8.40 g, 65.65 mmol) and a solution of potassium phosphate (15 g) in water (20 ml). This mixture is heated at reflux for 3 h. After cooling, water is added and the mixture is extracted with ethyl acetate (3 x 70 ml), dried (Na₂SO₄) and evaporated under reduced pressure. The residue is purified by column chromatography, eluting with petroleum ether, to give a brown oil (6.05 g, 84%). ¹H NMR (300 MHz, CDCl3): δ (ppm) 7.23 (dd, *J* = 5.2, 1.2 Hz, 1H, Ar-H), 7.14 (dd, *J* = 5.1, 1.2 Hz, 1H, Ar-H), 7.11 (m, 2H, Ar-H), 7.01 (m, 1H, Ar-H), 6.94 (m, 2H, Ar-H), 2.32 (s, 3H, CH₃); ¹³C NMR (75 MHz, CDCl₃): δ (ppm) 137.2,136.4,134.7, 134.6, 130.1, 128.0, 127.98, 127.6, 125.5, 125.2, 124.4, 123.7, 15.6.

### Step 7.2: 5,5''-Dibromo-3'-methyl-[2,2',5',2'']terthiophene

A mixture of 3'-methyl-[2,2',5',2"]terthiophene (2.20 g, 8.38 mmol) and N-bromosuccinimide (2.99 g, 16.80 mmol) in acetic acid (30 ml) is stirred in the dark for 1 h. The precipitate formed is filtered and washed with water, then recrystallised with toluene, to give pale yellow crystals (2.28 g, 65%). ¹H NMR (300 MHz, CDCl3): δ (ppm) 7.01 (d, *J* = 4.0 Hz, 1H, Ar-H), 6.95 (d, *J* = 3.7 Hz, 1H, Ar-H), 6.87 (m, 3H, Ar-H), 2.31 (s, 3H, CH₃); ¹³C NMR (75 MHz, CDCl₃): δ (ppm) 138.3, 137.5, 135.1, 134.1, 130.7, 130.3, 129.5, 128.0, 125.7, 123.8, 112.0, 111.2, 15.4.

### Step 7.3:

To a stirred solution of 5,5"-dibromo-3'-methyl-[2,2',5',2"]terthiophene (0.13 g, 0.31 mmol) in anhydrous THF (20 ml) is added tetrakis(triphenylphophine)palladium(0) (0.05 g) under nitrogen, with stirring. After 15 min, 2-{5-[6-(3-ethyl-oxetan-3-ylmethoxy)-hexyl]thiophen-2-yl}-4,4,5,5-tetramethyl-[1,3,2]dioxaborolane (0.38 g, 0.93 mmol) and a solution of potassium carbonate (0.50 g, 3.62 mmol) in water (5 ml) is added. This mixture is heated at reflux for 1.5 h. After cooling, water is added and the mixture is extracted with ethyl acetate (3 x 50 ml). The extracts are dried (Na₂SO₄) and evaporated under reduced pressure. The residue is dissolved in ether (2 ml) and cooled to 0 °C. The resulting crystals are filtered and washed with a little ether, to give an orange product (0.16 g, 64%). ¹H NMR (300 MHz, CDCl₃): δ (ppm) 6.95-7.05 (m, 7H, Ar-H), 6.69 (m, 2H, Ar-H), 4.45 (d, *J* = 5.9 Hz, 4H, OCH₂), 4.38 (d, *J* = 5.9 Hz, 4H, OCH₂), 3.52 (s, 4H, OCH₂), 3.45 (t, *J* = 6.4 Hz, 4H, OCH₂), 2.80 (t, *J* = 7.2 Hz, 4H, ArCH₂), 2.40 (s, 3H, Ar-CH₃), 1.67-1.78 (m, 8H, CH₂), 1.59 (m, 4H, CH₂), 1.40 (m, 8H, CH₂), 0.88 (t, *J* = 7.5 Hz, 6H, CH₃); ¹³C NMR (75 MHz, CDCl₃): δ (ppm) 145.5, 145.4, 137.5, 136.8, 135.2, 134.6, 134.52, 134.49, 134.4, 130.0, 127.8, 125.8, 124.9, 124.2, 123.6, 123.4, 123.3, 78.7, 73.4, 71.5, 43.4, 31.5, 30.1, 29.5, 28.9, 26.8, 25.9, 15.7, 8.3. m.p. (°C) K 36 S_{X} 65.4 N 70.9 I.

## Claims

1. Compounds of formula I
P-Sp-X-A¹-(Z¹-A²)ₘ-R I
wherein
P is a polymerisable group selected from CH₂=CW¹-COO-, 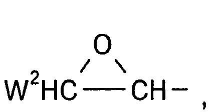 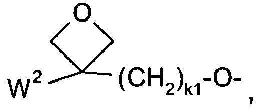 CH₂=CW²-(O)ₖ₁-, CH₃-CH=CH-O-, (CH₂=CH)₂CH-OCO-, (CH₂=CH-CH₂)₂CH-OCO-, (CH₂=CH)₂CH-O-, (CH₂=CH-CH₂)₂N-, (CH₂=CH-CH₂)₂N-CO-, HO-CW²W³-, HS-CW²W³-, HW²N-, HO-CW²W³-NH-, CH₂=CW¹-CO-NH-, CH₂=CH-(COO)ₖ₁-Phe-(O)ₖ₂-, Phe-CH=CH-, HOOC-, OCN-, and W⁴W⁵W⁶Si-, with W¹ being H, Cl, CN, phenyl or alkyl with 1 to 5 C-atoms, in particular H, Cl or CH₃, W² and W³ being independently of each other H or alkyl with 1 to 5 C-atoms, in particular methyl, ethyl or n-propyl, W⁴, W⁵ and W⁶ being independently of each other Cl, oxaalkyl or oxacarbonylalkyl with 1 to 5 C-atoms, Phe being 1,4-phenylene that is optionally substituted by one or more groups R¹, and k₁ and k₂ being independently of each other 0 or 1,
R¹ is H or alkyl or alkoxy with 1 to 15 C atoms which is optionally mono-, poly- or perfluorinated,
Sp is a linear or branched alkylene group having 1 to 20 C atoms in which, in addition, one or more non-adjacent CH₂ groups may be replaced by -O-, -S-,-NH-, -N(CH₃)-, -CO-, -O-CO-, -S-CO-, -O-CO-O-,-NR⁰-CO-NR⁰, -NR⁰-CO-O-, -O-CO-NR⁰-, -CO-S-,-CO-O-, -CH(halogen)-, -C(halogen)₂, -CH(CN)-,-CH=CH- or -C≡C-, or a siloxane group, and R⁰ is H or alkyl with 1 to 12 C-atoms,
X is -O-, -O-CH₂-, -CH₂-O- or a single bond,
A¹ and A² are unsubstituted thiophene-2,5-diyl,
Z¹ is a single bond,
R is P-Sp-X-, and
m is 4 or 5.

2. Compounds according to claim 1, selected from the following formulae 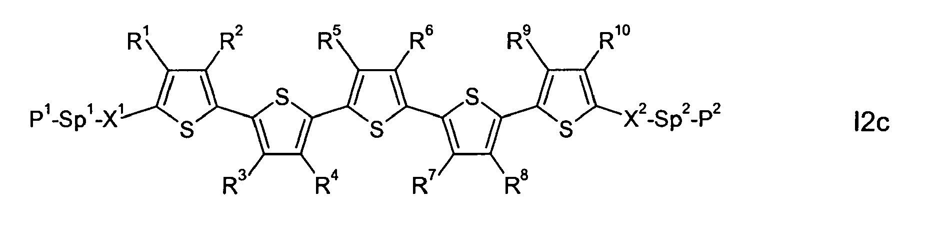 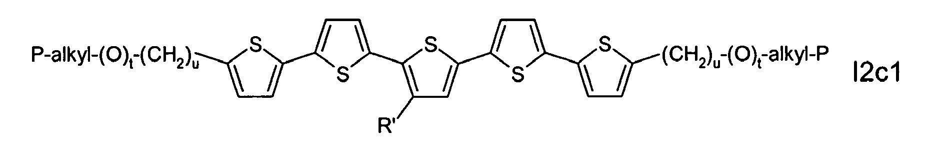 wherein
P¹ and P² are identical or different groups P as defined in claim 1,
Sp¹ and Sp² are identical or different groups Sp as defined in claim 1,
X¹ and X² are identical or different groups X as defined in claim 1,
R¹ to R⁸ are H,
"alkyl" is an alkylene group with 1 to 12 C-atoms,
t is 0 or 1,
u is 0 or 1,
R' is H,
P is CH₂=CW-COO-, 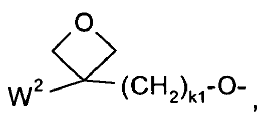 (CH₂=CH)₂CH-OCO- or (CH₂=CH-CH₂)₂N-CO-, W is H, CH₃, Cl or CN, and k1 is 0 or 1.

3. Reactive liquid crystal mixture comprising one or more compounds according to claim 1 or 2 and optionally one or more further reactive compounds, wherein at least one of said compounds is mesogenic or liquid crystalline.

4. Anisotropic polymer obtainable from a compound or mixture according to at least one of claims 1 to 3.

5. Anisotropic polymer with charge transport properties obtainable from one or more compounds or mixtures according to at least one of claims 1 to 3 that are aligned in their liquid crystal phase into macroscopically uniform orientation and polymerised or crosslinked to fix the oriented state.

6. Side chain liquid crystal polymer obtained by polymerisation of one or more compounds or mixtures according to at least one of claims 1 to 3 or by grafting the compounds or mixture components to a polymer backbone in a polymeranaloguous reaction, optionally with one or more additional mesogenic or non-mesogenic comonomers.

7. Use of the compounds, mixtures and polymers according to at least one of claims 1 to 6 as semiconductors or charge transport materials, in optical, electrooptical or electronic devices, components of integrated circuitry, field effect transistors (FET), thin film transistors in flat panel display applications, Radio Frequency Identification (RFID) tags, semiconducting components for organic light emitting diode (OLED) applications, electroluminescent displays, backlights of liquid crystal displays, photovoltaic or sensor devices, electrode materials in batteries, photoconductors, electrophotographic applications, electrophotographic recording, light-modulating components for liquid crystal displays, optical films or optical or electrooptical devices.

8. FET, component of integrated circuitry, thin film transistor, RFID tag, photovoltaic or sensor device, comprising one or more compounds, mixtures or polymers according to at least one of claims 1 to 6.

9. Security marking or device comprising comprising one or more compounds, mixtures or polymers according to at least one of claims 1 to 6, or a FET or RFID tag according to claim 8.

10. Compounds, mixtures or polymers according to at least one of claims 1 to 6, which are oxidatively or reductively doped to form conducting ionic species.

11. Charge injection layer, planarising layer, antistatic film or conducting substrate or pattern for electronic applications or flat panel displays, comprising one or more compounds, mixtures or polymers according to claim 10.
